# EUROPÄISCHE PATENTSCHRIFT

(11) **EP 1 542 953 B1**
(45) Veröffentlichungstag und Bekanntmachung des Hinweises auf die Patenterteilung: **05.12.2012**
(21) Anmeldenummer: 03722545.5
(22) Anmeldetag: 25.04.2003
(51) Int. Cl.: C07C 43/11, C07C 41/03, C08G 65/26, C11D 1/72, B01J 37/03, B01J 27/26

(54) **HERSTELLUNG VON ALKOXYLATEN BEI OPTIMIERTEN REAKTIONSDRÜCKEN**
METHOD FOR PRODUCING ALKOXYLATED PRODUCTS AT OPTIMIZED REACTION PRESSURES
REALISATION DE PRODUITS ALKOXYLES A DES PRESSIONS DE REACTION OPTIMISEES

(30) Priorität: 18.09.2002 DE 10243366
(43) Veröffentlichungstag der Anmeldung: 22.06.2005
(73) Patentinhaber: BASF SE, 67056 Ludwigshafen (DE)
(72) Erfinder: WULFF, Christian, 68163 Mannheim (DE); STÖSSER, Michael, 67141 Neuhofen (DE); GROSCH, Georg, Heinrich, 2930 Brasschaat (BE); BALDENIUS, Kai-Uwe, 67063 Ludwigshafen (DE); BOHRES, Edward, 68161 Mannheim (DE)
(86) Internationale Anmeldenummer: PCT/EP2003/004332
(87) Internationale Veröffentlichungsnummer: WO 2004/033404

(56) Entgegenhaltungen:
- WO-A-01/04183
- WO-A-94/11331

## Beschreibung

Die vorliegende Erfindung betrifft ein Verfahren zur Herstellung mindestens eines Alkoxylats, umfassend das Inkontaktbringen eines Alkylenoxidgemischs, mindestens enthaltend Ethylenoxid, mit mindestens einer Starterverbindung in Gegenwart mindestens einer Doppelmetallcyanid-Verbindung, wobei während der Induktionsphase die Summe aus Inertgas-Partialdruck und Ethylenoxid-Partialdruck bei 3,0 bar bis 6,0 bar liegt, und die Starterverbindung ein Guerbet-Alkohol ist.

Aus der Literatur ist bekannt, dass Doppelmetallcyanid-Verbindungen (DMC-Verbindungen) als Katalysatoren zur Umsetzung von Startermolekülen mit aktivem Wasserstoff und Alkylenoxiden, beispielsweise in einer Polymerisationsreaktion eingesetzt werden können. Die Ring-öffnende Polymerisationen von Alkylenoxiden wird beispielsweise in der EP-A 0 892 002, EP-A 0 862 977 und in der EP-A 0 755 716 beschrieben. DMC-Verbindungen weisen bei der Polymerisation von Alkylenoxiden eine hohe Aktivität als Katalysator auf.

Verfahren zur Alkoxylierung von aliphatischen Alkoholen sowie die erhaltenen Alkoxylate sind prinzipiell aus dem Stand der Technik bekannt. In der WO 01/04183 wird beispielsweise ein Verfahren zur Ethoxylierung von hydroxyfunktionellen Starterverbindungen beschrieben, das in Gegenwart einer Doppelmetallcyanid-Verbindung als Katalysator durchgeführt wird.

Alkoxylate von aliphatischen Alkoholen werden in großem Umfang als Tenside, Emulgatoren oder Schaumdämpfer eingesetzt. Die Benetzungs- und Emulgatoreigenschaften hängen dabei stark von der Art des Alkohols und der Art und Menge der Alkoxid-Addukte ab.

WO 94/11330 betrifft Alkoxylate von 2-Propylheptanol und deren Verwendung. In den Alkoxylaten liegt 2-Propylheptanol, zunächst mit 1 bis 6 mol Propylenoxid und sodann mit 1 bis 10 mol Ethylenoxid in Gegenwart von Alkalihydroxiden als Katalysator umgesetzt, vor. Gemäß den Beispielen wird ein zunächst mit 4 mol Propylenoxid und sodann mit 6 mol Ethylenoxid umgesetztes 2-Propylheptanol eingesetzt. Es wird angegeben, dass die Alkylenoxidaddukte ein verbessertes Verhältnis von Schaumverhalten zu Detergenzwirkung zeigen. Ferner ist angegeben, dass die Alkoxylate ein gutes Benetzungsverhalten zeigen. Sie werden in Detergenzzusammensetzungen zur Reinigung von Textilmaterialien eingesetzt. WO 94/11331 betrifft die Verwendung derartiger Alkoxylate.

US 2,508,036 betrifft ebenfalls die Verwendung von 2-n-Propylheptanolethoxylaten, die 5 bis 15 mol Ethylenoxid enthalten, als Netzmittel in wässrigen Lösungen. Es ist beschrieben, dass die Produkte als Tenside in Waschmitteln eingesetzt werden können.

Die DE -A- 102 18 754 sowie die DE -A- 102 18 753 betreffen die Verwendung von C₁₀-Alkanolalkoxylat-Gemischen, insbesondere Alkanolethoxylat-Gemische, derartige C₁₀-Alkanolalkoxylat-Gemische und Verfahren zu ihrer Herstellung. Die DE -A- 102 18 752 beschreibt ebenfalls Alkoxylat-Gemische und diese enthaltende Waschmittel wie auch Verfahren zur Herstellung der Alkoxylat-Gemische und die Verwendung des Waschmittels zum Waschen oder Reinigen von Textilien.

Bei der Alkoxylierung, insbesondere der Ethoxylierung, von Starterverbindungen in Gegenwart von Doppelmetallcyanid-Verbindungen treten insbesondere zwei Schwierigkeiten auf. Zum einen ist die Induktionsphase der Reaktion zum Teil sehr lang, was zu einer Verlängerung der Reaktionszeiten und zu erhöhten Kosten führt, zum anderen lässt die Aktivität des Katalysators während der Reaktion häufig langsam nach, bis keine ausreichende Reaktionsgeschwindigkeit mehr vorhanden ist.

Eine Aufgabe der vorliegenden Reaktion bestand daher darin, ein Verfahren zur Ethoxylierung von Starterverbindungen mit verbessertem Umsatz der Starterverbindung, verkürzter Induktionsphase und verbesserter Katalysator-Stabilität bereitzustellen.

Erfindungsgemäß wird diese Aufgabe gelöst durch ein Verfahren zur Herstellung mindestens eines Alkoxylats, umfassend das Inkontaktbringen eines Alkylenoxidgemischs, mindestens enthaltend Ethylenoxid, mit mindestens einer Starterverbindung in Gegenwart mindestens einer Doppelmetallcyanid-Verbindung der allgemeinen Formel I:

M¹ₐ[M²(CN)_{b}(A)_{c}]_{d ·} fM¹gXₙ · h(H₂O)· eL · kP (I),

in der
- M¹ mindestens ein Metallion, ausgewählt aus der Gruppe bestehend aus Zn²⁺, Fe²⁺, Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺ La³⁺, Ce³⁺, Ce⁴⁺, Eu³⁺, Ti³⁺, Ti⁴⁺, Ag⁺, Rh²⁺, Rh³⁺, Ru²⁺, Ru³⁺ ist,
- M² mindestens ein Metallion, ausgewählt aus der Gruppe bestehend aus Fe²⁺, Fe³⁺, Co²⁺, Co³⁺, Mn²⁺, Mn³⁺, V^{a+}, V⁵⁺, Cr²⁺, Cr³⁺, Rh³⁺, Ru²⁺, Ir³⁺ist,
- A und X unabhängig voneinander ein Anion, ausgewählt aus der Gruppe, bestehend aus Halogenid, Hydroxid, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,
- L ein mit Wasser mischbarer Ligand ist, ausgewählt aus der Gruppe, bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,
- k eine gebrochene oder ganze Zahl größer oder gleich Null ist, und
- P ein organischer Zusatzstoff ist,
- a, b, c, d, g und n so ausgewählt sind, dass die Elektroneutralität der Verbindung (I) gewährleistet ist, wobei c = 0 sein kann,
- e die Anzahl der Ligandenmoleküle eine gebrochenen oder ganze Zahl größer 0 oder 0 ist,
- f und h unabhängig voneinander eine gebrochene oder ganze Zahl größer 0 oder 0 sind,
wobei während der Induktionsphase die Summe aus Inertgas-Partialdruck und Ethylenoxid-Partialdruck bei 3,0 bar bis 6,0 bar liegt und die Starterverbindung ein Guerbet-Alkohol ist.

Unter einer Induktionsphase wird verstanden, dass die Alkoxylierungsreaktion nach dem Inkontaktbringen des Alkylenoxidgemisches mit dem Starteralkohol und der Doppelmetallcyanidverbindung nicht sofort beginnt, sondern um eine gewisse Zeit verzögert ist. Diese Induktionsphase äußerst sich beispielsweise dadurch, dass nach der Dosierung einer kleinen Menge Alkylenoxid ein gewisser Druck im Reaktor entsteht, der für eine gewisse Zeit konstant bleibt und am Ende der Induktionsphase schnell abfällt. Nach dem Druckabfall ist die Reaktion angesprungen, und die weitere Dosierung des Alkylenoxids kann erfolgen.

Erfindungsgemäß liegt die Summe aus Inertgas-Partialdruck und Ethylenoxid-Partialdruck während der Induktionsphase bei 3,0 bis 6,0 bar. Dieser Druckbereich ist besonders vorteilhaft, da zum einen ein schnelles Anspringen der Reaktion beobachtet wird, zum anderen jedoch der Druck nicht zu hoch ist. Der Gesamtdruck setzt sich aus den Partialdrücken der einzelnen Gase zusammen. Sofern nur Inertgas und Ethylenoxid eingesetzt werden, entspricht die Summe aus Inertgas-Partialdruck und Ethylenoxid-Partialdruck dem Gesamtdruck. Ein hoher Gesamtdruck zu Beginn der Reaktion würde beispielsweise teure Reaktionsgefäße erfordern und damit das gesamte Verfahren verteuern.

In einer bevorzugten Ausführungsform wird in dem erfindungsgemäßen Verfahren zunächst der Reaktor mit einem Inertgas beaufschlagt, und anschließend wird das Alkylenoxidgemisch mindestens enthaltend Ethylenoxid zugegeben. Der Inertgas-Partialdruck liegt erfindungsgemäß allgemein bei 1,5 bis 6,0 bar, vorzugsweise bei 1,5 bar bis 3,0 bar, bevorzugt bei 1,5 bis 2,5 bar, besonders bevorzugt bei 1,5 bis 2,0 bar.

Der Ethylenoxid-Partialdruck ist im Rahmen der vorliegenden Erfindung kleiner oder gleich dem Inertgas-Partialdruck, wobei die Summe aus Inertgas-Partialdruck und Ethylenoxid-Partialdruck bei 3,0 bar bis 6,0 bar liegt.

Diese Fahrweise hat den Vorteil, dass mit dem Inertgas ein Partialdruck eingestellt werden kann und anschließend das Alkylenoxidgemisch, mindestens enthaltend Ethylenoxid, zudosiert werden kann, wobei die Ethylenoxid-Konzentration in der Gasphase nicht zu hoch ist. Aus Sicherheitsgründen sollte eine Konzentration von >40%, bevorzugt >50% Ethylenoxid in der Gasphase im Reaktor vermieden werden, da es bei hohen Konzentrationen zum spontanen EO-Zerfall und damit einer Überhitzung oder Explosion des Reaktors kommen kann.

Der Ethylenoxid-Partialdruck wird bei einer derartigen Fahrweise im wesentlichen durch das Verhältnis der Zudosierung von Ethylenoxid und der Reaktionsgeschwindigkeit des Ethylenoxids bestimmt. Im Rahmen der vorliegenden Erfindung ist der Ethylenoxid-Partialdruck während der Induktionsphase vorzugsweise kleiner als 3,0 bar, insbesondere kleiner als 1,5 bar, beispielsweise kleiner als 1,0 bar.

Bei dem erfindungsgemäßen Verfahren kann beispielsweise zunächst das Reaktionsgefäß mit einer Suspension aus Alkohol und DMC-Katalysator befüllt werden. Anschließend kann der Katalysators durch Abtrennen von Wasser, z. B. durch Erhitzen und/oder Evakuieren des Reaktionsgefäßes aktiviert werden.

Anschließend wird das Reaktionsgemisch vorteilhafterweise auf Reaktionstemperatur erwärmt, und ein Stickstoff-Vordruck wird eingestellt. Im weiteren Verlauf des Verfahrens wird beispielsweise eine Startmenge Ethylenoxid zudosiert. Nach dem Anspringen der Reaktion wird weiteres Ethylenoxid zudosiert, das Reaktionsgemisch wird gerührt, bis alles Ethylenoxid abreagiert ist. Das Reaktionsgemisch kann gegebenenfalls weiter aufgearbeitet werden.

In einer bevorzugten Ausführungsform betrifft die vorliegende Erfindung daher ein Verfahren, wobei während der Induktionsphase der Inertgas-Partialdruck bei 1,5 bar bis 6,0 bar, bevorzugt bei 1,5 bis 3,0 bar liegt. Geeignete Inertgase sind im Rahmen der vorliegenden Erfindung beispielweise Stickstoff, CO₂ oder Edelgase wie Argon oder Gemische davon, bevorzugt Stickstoff.

Erfindungsgemäß ändert sich der Druck während der Reaktion. Nach einem möglichen anfänglichen Druckabfall beim Anspringen der Reaktion steigt der Druck im Verlauf der Reaktion, da der Füllstand im Reaktor steigt und das Gasgemisch komprimiert wird. Erfindungsgemäß ist es bevorzugt, dass der Gesamtdruck, insbesondere die Summe aus Inertgas-Partialdruck und Ethylenoxid-Partialdruck, im Verlauf der Umsetzung nicht über 20 bar, beispielsweise nicht über 11 bar, vorzugsweise nicht über 6 bar, insbesondere nicht über 3,5 bar steigt.

In einer bevorzugten Ausführungsform betrifft die vorliegende Erfindung daher ein Verfahren, wobei der Gesamtdruck im Verlauf der Umsetzung nicht über 11 bar steigt.

Das erfindungsgemäße Verfahren wird bei Temperaturen von 80 bis 190°C durchgeführt.

Das erfindungsgemäßen Verfahren zur Herstellung eines Alkoxylats wird in Gegenwart einer Doppelmetallcyanid-Verbindung der allgemeinen Formel I als Katalysator durchgeführt:

M¹ₐ[M²(CN)_{b}(A)_{c}]_{d} fM¹_{g}Xₙ· h(H₂O)· eL·kP (I),

in der
- M¹ mindestens ein Metallion, ausgewählt aus der Gruppe bestehend aus Zn²⁺, Fe²⁺, Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺ ist,
- M² mindestens ein Metallion, ausgewählt aus der Gruppe bestehend aus Fe²⁺, Fe³⁺, CO²⁺, CO³⁺, Mn²⁺, Mn³⁺, V⁴⁺, V⁵⁺, Cr²⁺, Cr³⁺, Rh³⁺, Ru²⁺, Ir³⁺ ist,
- A und X unabhängig voneinander ein Anion, ausgewählt aus der Gruppe, bestehend aus Halogenid, Hydroxid, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,
- L ein mit Wasser mischbarer Ligand ist, ausgewählt aus der Gruppe, bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,
- k eine gebrochene oder ganze Zahl größer oder gleich Null ist, und
- P ein organischer Zusatzstoff ist,
- a, b, c, d, g und n so ausgewählt sind, dass die Elektroneutralität der Verbindung (I) gewährleistet ist, wobei c = 0 sein kann,
- e die Anzahl der Ligandenmoleküle eine gebrochenen oder ganze Zahl größer 0 oder 0 ist,
- f und h unabhängig voneinander eine gebrochene oder ganze Zahl größer 0 oder 0 sind.

Als organische Zusatzstoffe P sind zu nennen: Polyether, Polyester, Polycarbonate, Polyalkylenglykolsorbitanester, Polyakylenglykolglycidylether, Polyacrylamid, Poly(acrylamid-co-acrylsäure), Polyacrylsäure, Poly(acrylamid-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly(acrylsäure-co-styrol), Oxazolinpolymere, Polyalkylenimine, Maleinsäure- und Maleinsäureanhydridcopolymere, Hydroxyethylcellulose, Polyacetate, ionische oberflächen- und grenzflächenaktive Verbindungen, Gallensäure oder deren Salze, Ester oder Amide, Carbonsäureester mehrwertiger Alkohole und Glycoside.

Diese Katalysatoren können kristallin oder amorph sein. Für den Fall, dass k gleich null ist, sind kristalline Doppelmetallcyanid-Verbindungen bevorzugt. Im Fall, dass k größer null ist, sind sowohl kristalline, teilkristalline, als auch substantiell amorphe Katalysatoren bevorzugt.

Von den modifizierten Katalysatoren gibt es verschiedene bevorzugte Ausführungsformen. Eine bevorzugte Ausführungsform sind Katalysatoren der Formel (I), bei denen k größer null ist. Der bevorzugte Katalysator enthält dann mindestens eine Doppelmetallcyanid-Verbindung, mindestens einen organischen Liganden und mindestens einen organischen Zusatzstoff P.

Bei einer anderen bevorzugten Ausführungsform ist k gleich null, optional ist e auch gleich null und X ist ausschließlich ein Carboxylat, bevorzugt Formiat, Acetat und Propionat. Derartige Katalysatoren sind in der WO 99/16775 beschrieben. Bei dieser Ausführungsform sind kristalline Doppelmetallcyanid-Katalysatoren bevorzugt. Ferner bevorzugt sind Doppelmetallcyanid-Katalysatoren, wie in der WO 00/74845 beschrieben, die kristallin und plättchenförmig sind.

In einer bevorzugten Ausführungsform betrifft die vorliegende Erfindung daher ein Verfahren, bei dem die als Katalysator eingesetzte Doppelmetallcyanid-Verbindung kristallin ist.

Die Herstellung der modifizierten Katalysatoren erfolgt durch Vereinigung einer Metallsalz-Lösung mit einer Cyanometallat-Lösung, die optional sowohl einen organischen Liganden L als auch einen organischen Zusatzstoff P enthalten können. Anschließend werden der organische Ligand und optional der organische Zusatzstoff zugegeben. Bei einer bevorzugten Ausführungsform der Katalysatorherstellung wird zunächst eine inaktive Doppelmetallcyanid-Phase hergestellt und diese anschließend durch Umkristallisation in eine aktive Doppelmetallcyanidphase überführt, wie in der PCT/EP01/01893 beschrieben.

Bei einer anderen bevorzugten Ausführungsform der Katalysatoren sind f, e und k ungleich Null. Dabei handelt es sich um Doppelmetallcyanid-Katalysatoren, die einen mit Wasser mischbaren organischen Ligand (im allgemeinen in Mengen von 0,5 bis 30 Gew.%) und einen organischen Zusatzstoff (im allgemeinen in Mengen von 5 bis 80 Gew.%) enthalten wie in der WO 98/06312 beschrieben. Die Katalysatoren können entweder unter starkem Rühren (24000U/Min mit Turrax) oder unter Rühren hergestellt werden wie in der US 5,158,922 beschrieben.

Insbesondere als Katalysator geeignet sind für die Alkoxylierung Doppelmetallcyanid-Verbindungen, die Zink, Kobalt oder Eisen oder zwei davon enthalten. Besonders geeignet ist beispielsweise Berliner Blau.

Bevorzugt werden kristalline DMC-Verbindungen eingesetzt. In einer bevorzugten Ausführungsform wird eine kristalline DMC-Verbindung vom Zn-Co-Typ als Katalysator verwendet, der als weitere Metallsalzkomponente Zinkacetat enthält. Derartige Verbindungen kristallisieren in monokliner Struktur und weisen einen plättchenförmigen Habitus auf. Derartige Verbindungen werden beispielsweise in der WO 00/74845 oder der PCT/EP01/011893 beschrieben.

Als Katalysator geeignete DMC-Verbindungen können prinzipiell auf alle dem Fachmann bekannten Arten hergestellt werden. Beispielsweise können die DMC-Verbindungen durch direkte Fällung, "incipient wetness"-Methode, durch Herstellung einer Precursor-Phase und anschließende Umkristallisation hergestellt werden.

Die DMC-Verbindungen können als Pulver, Paste oder Suspension eingesetzt werden oder zu einem Formkörper verformt werden, in Formkörpern, Schäume oder ähnliches eingebracht werden oder auf Formkörper, Schäume oder ähnliches aufgebracht werden.

Die zur Alkoxylierung eingesetzte Katalysator-Konzentration bezogen auf das Endmengengerüst ist typischerweise kleiner als 2000 ppm (d.h. mg Katalysator pro kg Produkt), bevorzugt kleiner als 1000 ppm, insbesondere kleiner als 500 ppm, besonders bevorzugt kleiner als 100 ppm, beispielsweise kleiner als 50 ppm oder 35 ppm, insbesondere bevorzugt kleiner als 25 ppm.

In einer bevorzugten Ausführungsform betrifft die vorliegende Erfindung ein Verfahren, bei dem die Doppelmetallcyanid-Verbindung in einer Menge von 100 ppm oder weniger bezogen auf das Endmengengerüst eingesetzt wird.

In weiteren Ausführungsformen betrifft die vorliegende Erfindung ein Verfahren, wobei mindestens eine der folgenden Eigenschaften erfüllt ist:
(1) M¹ ist ausgewählt aus der Gruppe Zn²⁺, Fe²⁺, Fe³⁺, Co³⁺, Ni²⁺, Mn²⁺, Co²⁺;
(2) M² ist ausgewählt aus der Gruppe Fe²⁺, Fe³⁺, Co³⁺.
oder besonders bevorzugt ein Verfahren, wobei M¹ Zn²⁺ ist und M² Co³⁺.

Bei den erfindungsgemäß als Starterverbindung eingesetzten Alkoholen handelt es sich um Guerbet-Alkohole, insbesondere Ethylhexanol, Propylheptanol, Butyloctanol.

Bei den als Starterverbindung eingesetzten Alkoholen kann es sich erfindungsgemäß auch um Gemische verschiedener Isomere handeln. Beispielsweise kann Propylheptanol ausgehend von Valeraldehyd durch Aldolkondensation und nachfolgende Hydrierung erhalten werden. Die Herstellung von Valeraldehyd und den entsprechenden Isomeren erfolgt durch Hydroformylierung von Buten, wie beispielsweise in US 4,287,370; Beilstein E IV 1, 32 68, Ullmanns Encyclopedia of Industrial Chemistry, 5. Auflage, Band A1, Seiten 323 und 328 f beschrieben. Die nachfolgende Aldolkondensation ist beispielsweise beschrieben in US 5,434,313 und Römpp, Chemie Lexikon, 9. Auflage, Stichwort "Aldol-Addition" Seite 91. Die Hydrierung des Aldolkondensationsproduktes folgt allgemeinen Hydrierbedingungen.

Des weiteren kann 2-Propylheptanol durch Kondensation von 1-Pentanol (als Mischung der entsprechenden Methylbutanole-1) in Gegenwart von KOH bei erhöhten Temperaturen hergestellt werden, siehe z.B. Marcel Guerbet, C.R Acad Sci Paris 128, 511, 1002 (1899). Des weiteren ist auf Römpp, Chemie Lexikon, 9. Auflage, Georg Thieme Verlag Stuttgart, und die dort genannten Zitate sowie Tetrahedron, Vol. 23, Seiten 1723 bis 1733, hinzuweisen.

Das im erfindungsgemäßen Verfahren eingesetzte Alkylenoxidgemisch kann neben Ethylenoxid weitere Alkylenoxide enthalten, insbesondere ein weiteres Alkylenoxid ausgewählt aus der Gruppe bestehend aus Propylenoxid, Butylenoxid und Pentylenoxid.

Dabei haben die Alkylenoxidgemische im Rahmen der vorliegenden Erfindung bevorzugt einen Ethylenoxid-Anteil von mehr als 50 % (Massen-%), insbesondere von mehr als 75%, besonders bevorzugt von mehr als 95%, beispielsweise von mehr als 99%. In einer bevorzugten Ausführungsform wird erfindungsgemäß neben Ethylenoxid kein weiteres Alkylenoxid eingesetzt.

Daher betrifft die vorliegende Erfindung in einer weiteren Ausführungsform ein Verfahren, wobei das Alkylenoxidgemisch Ethylenoxid und ein weiteres Alkylenoxid ausgewählt aus der Gruppe bestehend aus Propylenoxid, Butylenoxid und Pentylenoxid enthält.

In einer weiteren Ausführungsform betrifft die vorliegende Erfindung somit auch ein Verfahren, bei dem neben Ethylenoxid kein weiteres Alkylenoxid eingesetzt wird.

Vorzugsweise wird das Alkylenoxidgemisch im erfindungsgemäßen Verfahren in solchen Mengen eingesetzt, das der erhaltene Alkoxylierungsgrad beispielsweise im Bereich von 2 bis 20, bevorzugt im Bereich von 3 bis 14 liegt.

Die erfindungsgemäß hergestellten Alkoxylate zeigen eine gute Netzung auf harten Oberflächen. Das vorteilhafte Netzungsverhalten der erfindungsgemäßen Gemische kann beispielsweise durch Messungen des Kontaktwinkels auf Glas, Polyethylenoxid oder Stahl ermittelt werden. Die erfindungsgemäßen Alkoxylate zeigen außerdem ein gutes Emulgierverhalten kombiniert mit leichter biologischer Abbaubarkeit.

Die erfindungsgemäß hergestellten Alkoxylate, insbesondere Ethoxylat, können als Emulgator, Schaumregulierer und als Netzmittel für harte Oberflächen, insbesondere in Waschmitteln, Tensidformulierungen zur Reinigung harter Oberflächen, Feuchthaltemitteln, kosmetischen, pharmazeutischen und Pflanzenschutzformulierungen, Lacken, Beschichtungsmitteln, Klebstoffen, Lederentfettungsmitteln, Formulierungen für die Textilindustrie, Faserverarbeitung, Metallverarbeitung, Lebensmittelindustrie, Wasserbehandlung, Papierindustrie, Fermentation oder Mineralverarbeitung und in Emulsionspolymerisationen verwendet werden.

Ferner dienen die erfindungsgemäß hergestellten Alkoxylate zur Verminderung der Grenzflächenspannung, beispielsweise in wässrigen Tensidformulierungen. Die verminderte Grenzflächenspannung kann beispielsweise durch die Pendant-Drop-Methode bestimmt werden. Hieraus ergibt sich auch eine bessere Wirkung der erfindungsgemäßen Alkoxylate als Emulgator oder Co-Emulgator. Die erfindungsgemäßen Alkoxylate können auch zur Verminderung der Grenzflächenspannung bei kurzen Zeiten von üblicherweise unter einer Sekunde bzw. zur Beschleunigung der Einstellung der Grenzflächenspannung in wässrigen Tensidformulierungen eingesetzt werden.

Nachstehend werden bevorzugte Einsatzgebiete der erfindungsgemäß hergestellten Alkoxylate näher beschrieben.

Die erfindungsgemäß hergestellten Alkoxylate werden vorzugsweise in den folgenden Bereichen eingesetzt:
- Tensidformulierungen zur Reinigung harter Oberflächen: Geeignete Tensidformulierungen, die mit den erfindungsgemäßen Alkoxylaten additiviert werden können, sind beispielsweise in Formulating Detergents and Personal Care Products von Louis Ho Tan Tai, AOCS Press, 2000 beschrieben.

Sie enthalten beispielsweise als weitere Komponenten Seife, anionische Tenside wie LAS oder Paraffinsulfonate oder FAS oder FAES, Säure wie Phosphorsäure, Amidosulfonsäure, Zitronensäure, Milchsäure, Essigsäure, andere organische und anorganische Säuren, Lösungsmittel wie Ethylenglykol, Isopropanol, Komplexbildner wie EDTA, NTA, MGDA, Phosphonate, Polymere wie Polyacrylate, Copolymere Maleinsäure-Acrylsäure, Alkalispender wie Hydroxide, Silicate, Carbonate, Parfümöle, Oxidationsmittel wie Perborate, Persäuren oder Trichloroisocyanursäure, Na- oder K-Dichlorisocyanurate, Enzyme; siehe auch Milton J. Rosen, Manilal Dahanayake, Industrial Utilization of Surfactants, AOCS Press, 2000 und Nikolaus Schönfeldt, Grenzflächenaktive Ethylenoxidaddukte. Hier sind auch Formulierungen für die anderen genannten Anwendungen im Prinzip abgehandelt. Es kann sich um Haushaltsreiniger wie Allzweckreiniger, Geschirrspülmittel für manuelles wie automatisches Geschirrspülen, Metallentfettung, Industrielle Applikationen wie Reinigungsmittel für die Nahrungsmittelindustrie Flaschenwäsche, etc. handeln. Es kann sich auch um Druckwalzen- und Druckplattenreinigungsmittel in der Druckindustrie handeln. Geeignete weitere Inhaltsstoffe sind dem Fachmann bekannt.
- Feuchthaltemittel, insbesondere für die Druckindustrie.
- Kosmetische, pharmazeutische und Pflanzenschutzformulierungen. Geeignete Pflanzenschutzformulierungen sind beispielsweise in der EP-A-0 050 228 beschrieben. Es können für Pflanzenschutzmittel übliche weitere Inhaltsstoffe vorliegen.
- Lacke, Beschichtungsmittel, Farben, Pigmentpräparationen sowie Klebstoffe in der Lack- und Folienindustrie.
- Lederentfettungsmittel.
- Formulierungen für die Textilindustrie wie Egalisiermittel oder Formulierungen zur Garnreinigung.
- Faserverarbeitung und Hilfsmittel für die Papier- und Zellstoffindustrie.
- Metallverarbeitung wie Metallveredelung und Galvanobereich.
- Lebensmittelindustrie.
- Wasserbehandlung und Trinkwassergewinnung.
- Fermentation.
- Mineralverarbeitung und Staubkontrolle.
- Bauhilfsmittel.
- Emulsionspolymerisation und Herstellung von Dispersionen.
- Kühl- und Schmiermittel.

Solche Formulierungen enthalten üblicherweise Inhaltsstoffe wie Tenside, Gerüst-, Duft - und Farbstoffe, Komplexbildner, Polymere und andere Inhaltsstoffe. Typische Formulierungen sind beispielsweise in WO 01/32820 beschrieben. Weitere für unterschiedliche Anwendungen geeignete Inhaltsstoffe sind in EP-A-0 620 270, WO 95/27034, EP-A-0 681 865, EP-A-0 616 026, EP-A-0 616 028, DE-A-42 37 178 und US 5,340,495 und in Schönfeldt, s.o., beispielhaft beschrieben.

Allgemein können die erfindungsgemäß hergestellten Alkoxylate in allen Bereichen eingesetzt werden, in denen die Wirkung von grenzflächenaktiven Stoffen notwendig ist.

Im folgenden soll die vorliegende Erfindung anhand von Beispielen näher erläutert werden.

### BEISPIELE

### Herstellbeispiel: Doppelmetallcyanid-Katalysator

In einem Rührkessel mit einem Volumen von 30 1, ausgestattet mit einem Propellerrührer, Tauchrohr für die Dosierung, pH-Sonde und Streulicht-Sonde, wurden 16000 g wässrige Hexacyanocobaltsäure (Cobalt-Gehalt: 9 g/l) vorgelegt und unter Rühren auf 50°C erwärmt. Anschließend wurden unter Rühren mit einer Rührleistung von 0,4 W/1 9224 g wässrige Zinkacetat-Dihydrat-Lösung (Zink-Gehalt: 2,6 Gew.-%), welche auf ebenfalls 50°C temperiert war, innerhalb von 15 Minuten zugefahren.

Zu dieser Fällsuspension wurden 351 g Pluronic® PE 6200 (BASF AG) zugesetzt und die Mischung weitere 10 Minuten gerührt.

Anschließend wurden weitere 3690 g wässrige Zinkacetat-Dihydrat-Lösung (Zink-Gehalt: 2,6 Gew.-%) unter Rühren mit einer Rührenergie von 1 W/1 innerhalb 5 Minuten zudosiert. Die Suspension wurde zwei Stunden nachgerührt. Der pH-Wert fiel in dieser Zeit von 4,02 auf 3,27 und blieb dann konstant. Die so erhaltene Fällsuspension wurde anschließend abfiltriert und auf dem Filter mit dem 6-fachen Kuchenvolumen an Wasser gewaschen.

Der feuchte Filterkuchen wurde getrocknet und mittels Spalt-Rotor-Mühle in Tridekanol® N dispergiert. Die dabei erhaltene Suspension hatte einen Multimetallcyanidgehalt von 5 Gew.%.

### Beispiel 1 (Vergleichsbeispiel, 2-Propylheptanol + 8 EO bei max. 1,70 bar Gesamtdruck)

316 g (2,0 Mol) 2-Propyl-Heptanol-1 (Isomerengemisch aus 87% 2-Propylheptanol-1, 11% 2-Propyl-4-methylhexanol-1, <1% 2-Propyl-5-methylhexanol-1) und 35 ppm Doppelmetallcyanid-Katalysator (bezogen auf das Produkt) wurden bei einer Temperatur von 100 °C und ca. 20 mbar für zwei Stunden in einem 3,5-1-Druckautoklaven entwässert. Anschließend wurde dreimal mit Stickstoff gespült und eine Temperatur von 140 °C eingestellt. Nach Erreichen der Temperatur sollten unter Rühren insgesamt 704 g (16,0 Mol) Ethylenoxid bei einem maximalen Gesamtdruck von 1,70 bar (absolut) zudosiert werden. Nach Zugabe von 582 g Ethylenoxid war keine Reaktion mehr feststellbar (kaum Druckabnahme, kaum Wärmeentwicklung).

### Beispiel 2: Anspringen der Reaktion bei Druckerhöhung

Die Reaktionsmischung aus Beispiel 1 wurde weiter gerührt und der Gesamtdruck durch Ethylenoxid-Zugabe auf 6,0 bar erhöht. Es waren wieder eine ausreichende Druckabnahme und Temperaturerhöhung feststellbar, so daß die restliche Menge an Ethylenoxid erfolgreich zur Reaktion gebracht werden konnte.

Nach beendeter Ethylenoxid-Dosierung rührte man noch 1 h bei 140 °C, kühlte danach auf 80 °C ab, spülte dreimal mit Stickstoff, evakuierte dann zum Entgasen auf 20 mbar, und entleerte den Reaktor. Das Reaktionsprodukt wurde nicht filtriert und entsprach dem gewünschten Produkt.

### Beispiel 3 (Vergleichsbeispiel, 2-Propylheptanol + 1,2 PO + 6 EO bei max. 1,70 bar EO-Druck)

316 g (2,0 Mol) 2-Propyl-Heptanol-1 und 35 ppm Doppelmetallcyanid-Katalysator (bezogen auf das Produkt) wurden bei einer Temperatur von 100 °C und ca. 20 mbar für zwei Stunden in einem Druckautoklaven entwässert. Anschließend wurde dreimal mit Stickstoff gespült und danach auf 140 °C erhitzt. Nach Erreichen der Temperatur wurden unter Rühren insgesamt 140 g (2,4 Mol) Propylenoxid bei 140 °C zudosiert. Nach Ende der PO-Dosierung rührte man noch 15 Minuten bei 140 °C.

Man hielt die Temperatur bei 140 °C, und es wurden anschliessend 528 g (12,0 Mol) Ethylenoxid bei einem maximalen Gesamtdruck von 1,70 bar zudosiert. Nach Zugabe von 424 g Ethylenoxid war keine ausreichende Reaktion mehr feststellbar (kaum Druckabnahme, kaum Wärementwicklung).

### Beispiel 4: Abspringen der Reaktion bei Druckerhöhung

Die Reaktionsmischung aus Beispiel 3 wurde weiter gerührt und der Gesamtdruck durch Ethylenoxid-Zugabe auf 6,0 bar erhöht. Es waren wieder eine ausreichende Druckabnahme und Temperaturerhöhung feststellbar, so daß die restliche Menge an Ethylenoxid erfolgreich zur Reaktion gebracht werden konnte.

Nach beendeter Ethylenoxid-Dosierung rührte man noch 1 h bei 140 °C, kühlte danach auf 80 °C ab, spülte dreimal mit Stickstoff, evakuierte dann zum Entgasen auf 20 mbar, und entleerte den Reaktor. Das Reaktionsprodukt wurde nicht filtriert und entsprach dem gewünschten Produkt.

### Beispiel 5 (2-Propylheptanol + 8 EO bei max. 2,5 bar EO-Druck)

316 g (2,0 Mol) 2-Propyl-Heptanol-1 und 35 ppm Doppelmetallcyanid-Katalysator (bezogen auf das Produkt) wurden bei einer Temperatur von 100 °C und ca. 20 mbar für zwei Stunden in einem Druckautoklaven entwässert. Anschließend wurde dreimal mit Stickstoff gespült und danach ein Gesamtdruck von 2,5 bar Stickstoff (absolut) bei 140 °C eingestellt. Nach Erreichen der Temperatur wurden unter Rühren insgesamt 704 g (16,0 Mol) Ethylenoxid bei einem Gesamtdruck von maximal 5,0 bar (absolut) zudosiert. Nach beendeter Ethylenoxid-Dosierung rührte man noch 1 h bei 140 °C, kühlte danach auf 80 °C ab, spülte dreimal mit Stickstoff, evakuierte dann zum Entgasen auf 20 mbar, und entleerte den Reaktor. Das Reaktionsprodukt wurde nicht filtriert und entsprach dem gewünschten Produkt.

### Beispiel 6 (2-Propylheptanol + 1,2 PO + 6 EO bei max. 2,5 bar EO-Druck)

316 g (2,0 Mol) 2-Propyl-Heptanol-1 (Isomerengemisch aus 87% 2-Propylheptanol-1, 11% 2-Propyl-4-methylhexanol-1, <1% 2-Propyl-5-methylhexanol-1) und 35 ppm Doppelmetallcyanid-Katalysator (bezogen auf das Produkt) wurden bei einer Temperatur von 100 °C und ca. 20 mbar für zwei Stunden in einem Druckautoklaven entwässert. Anschließend wurde dreimal mit Stickstoff gespült und danach auf 140 °C erhitzt. Nach Erreichen der Temperatur wurden unter Rühren insgesamt 140 g (2,4 Mol) Propylenoxid bei 140 °C zudosiert. Nach Ende der PO-Dosierung rührte man noch 15 Minuten bei 140 °C.

Dann stellte man einen Gesamtdruck von 2,5 bar Stickstoff (absolut) bei 140 °C ein und begann danach mit der Dosierung von insgesamt 528 g (12,0 Mol) Ethylenoxid bei einem Gesamtdruck von maximal 5,0 bar (absolut, 140 °C). Nach beendeter Ethylenoxid-Dosierung rührte man noch 1 h bei 140 °C, kühlte danach auf 80 °C ab, spülte dreimal mit Stickstoff, evakuierte dann zum Entgasen auf 20 mbar, und entleerte den Reaktor. Das Reaktionsprodukt wurde nicht filtriert und entsprach dem gewünschten Produkt.

## Patentansprüche

1. Verfahren zur Herstellung mindestens eines Alkoxylats, umfassend das Inkontaktbringen eines Alkylenoxidgemisches, mindestens enthaltend Ethylenoxid, mit mindestens einer Starterverbindung in Gegenwart mindestens einer Doppelmetallcyanid-Verbindung der allgemeinen Formel I:
M¹a[M²(CN)_{b}(A)_{c}]_{d} · fM¹gXₙ · h(H₂O) · eL · kP (I),
in der
- M¹ mindestens ein Metallion, ausgewählt aus der Gruppe bestehend aus Zn²⁺, Fe²⁺, Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺ ist,
- M² mindestens ein Metallion, ausgewählt aus der Gruppe bestehend aus Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺ ist,
- A und X unabhängig voneinander ein Anion, ausgewählt aus der Gruppe, bestehend aus Halogenid, Hydroxid, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,
- L ein mit Wasser mischbarer Ligand ist, ausgewählt aus der Gruppe, bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,
- k eine gebrochene oder ganze Zahl größer oder gleich Null ist, und neue Seite
- P ein organischer Zusatzstoff ist,
- a, b, c, d, g und n so ausgewählt sind, dass die Elektroneutralität der Verbindung (I) gewährleistet ist, wobei c = 0 sein kann,
- e die Anzahl der Ligandenmoleküle eine gebrochenen oder ganze Zahl größer 0 oder 0 ist,
- f und h unabhängig voneinander eine gebrochene oder ganze Zahl größer 0 oder 0 sind,
**dadurch gekennzeichnet, dass** während der Induktionsphase die Summe aus Inertgas-Partialdruck und Ethylenoxid-Partialdruck bei 3,0 bar bis 6,0 bar liegt und die Starterverbindung ein Guerbet-Alkohol ist.

2. Verfahren nach Anspruch 1, **dadurch gekennzeichnet, dass** der Gesamtdruck im Verlauf der Umsetzung nicht über 11 bar steigt.

3. Verfahren nach einem der Ansprüche 1 und 2, **dadurch gekennzeichnet, dass** mindestens eine der folgenden Eigenschaften erfüllt ist:
(1) M¹ ist ausgewählt aus der Gruppe Zn²⁺, Fe²⁺, Fe³⁺, Co³⁺, Ni²⁺, Mn²⁺, Co²⁺;
(2) M² ist ausgewählt aus der Gruppe Fe²⁺, Fe³⁺, Co³⁺.

4. Verfahren nach einem der Ansprüche 1 bis 3, **dadurch gekennzeichnet, dass** M¹ Zn²⁺ und M² Co³⁺ ist.

5. Verfahren nach einem der Ansprüche 1 bis 4, **dadurch gekennzeichnet, dass** die als Katalysator eingesetzte Doppelmetallcyanid-Verbindung kristallin ist

6. Verfahren nach einem der Ansprüche 1 bis 5, **dadurch gekennzeichnet, dass** das Alkylenoxidgemisch einen Ethylenoxid-Anteil von mehr als 99% aufweist.

## Claims

1. A process for the preparation of at least one alkoxylate, comprising the bringing into contact of an alkylene oxide mixture, at least comprising ethylene oxide, with at least one starter compound in the presence of at least one double-metal cyanide compound of the formula I:
M¹ₐ[M²(CN)_{b}(A)_{c}]_{d} · fM¹_{g}Xₙ ·h(H₂O) · eL · kP (I)
in which
- M¹ is at least one mental ion chosen from the group consisting of 2n²⁺, fe²⁺, Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺,
- M² is at least one metal ion chosen from the group consisting of Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺,
- A and X, independently of one another, are an anion chosen from the group consisting of halide, hydroxide, sulfate, carbonate, cyanide, thiocyanate, isocyanate, cyanate, carboxylate, oxalate, nitrate, nitrosyl, hydrogensulfate, phosphate, dihydrogenphosphate, hydrogenphosphate or hydrogencarbonate,
- L is a water-miscible ligand chosen from the group consisting of alcohols, aldehydes, ketones, ethers, polyethers, esters, polyesters, polycarbonate, ureas, amides, primary, secondary and tertiary amines, ligands with pyridine nitrogen, nitriles, sulfides, phosphides, phosphites, phosphines, phosphonates and phosphates,
- k is a fraction or integer greater than or equal to zero, and
- P is an organic additive,
- a, b, c, d, g and n are chosen such that the electroneutrality of the compound (I) is ensured, where c may be 0,
- e is the number of ligand molecules, a fraction or integer greater than 0, or 0,
- f and h, independently of one another, are a fraction or integer greater than 0, or 0,
wherein, during the induction phase, the sum of inert gas partial pressure and ethylene oxide partial pressure is 3.0 bar to 6.0 bar and the starter compound is a Guerbet alcohol.

2. The process according to claim 1, wherein the total pressure does not exceed 11 bar over the course of the reaction.

3. The process according to either of claims 1 and 2, wherein at least one of the following properties is satisfied:
(1) M¹ is chosen from the group Zn²⁺ Fe²⁺, Fe³⁺, Co³⁺, Ni²⁺, Mn²⁺, Co²⁺;
(2) M² is chosen from the group Fe²⁺, Fe³⁺, Co³⁺.

4. The process according to any of claims 1 to 3, wherein M¹ is Zn²⁺ and M² is Co³⁺.

5. The process according to any of claims 1 to 4, wherein the double-metal cyanide compound used as catalyst is crystalline.

6. The process according to any of claims 1 to 5, wherein the alkylene oxide mixture has an ethylene oxide fraction of more than 99%.

## Revendications

1. Procédé pour la préparation d'au moins un produit d'alcoxylation, comprenant la mise en contact d'un mélange d'oxydes d'alkylène, au moins contenant de l'oxyde d'éthylène, avec au moins un composé de départ en présence d'au moins un composé cyanure bimétallique de formule générale I :
M¹ₐ[M²(CN)_{b}(A)_{c}]_{d}·fM¹_{g}Xₙ·h(H₂O)·eL·kP (I)
dans laquelle
- M¹ est au moins un ion métallique, choisi dans le groupe constitué par Zn²⁺, Fe²⁺, Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺,
- M² est au moins un ion métallique, choisi dans le groupe constitué par Fe²⁺, Fe³⁺, Co²⁺, Co³⁺, Mn²⁺, Mn³⁺, V⁵⁺, Cr²⁺, Cr³⁺, Rh³⁺, Ru²⁺, Ir³⁺,
- A et X représentent, indépendamment l'un de l'autre, un anion choisi dans le groupe constitué par un ion halogénure, hydroxy, sulfate, carbonate, cyanure, thiocyanate, isocyanate, cyanate, carboxylate, oxalate, nitrate, nitrosyle, hydrogénosulfate, phosphate, dihydrogénophosphate, hydrogénophosphate ou hydrogénocarbonate,
- L est un ligand miscible à l'eau, choisi dans le groupe constitué par des alcools, des aldéhydes, des cétones, des éthers, des polyéthers, des esters, des polyesters, le polycarbonate, des urées, des amides, des amines primaires, secondaires et tertiaires, des ligands avec l'atome d'azote de la pyridine, des nitriles, des sulfures, des phosphures, des phosphites, des phosphanes, des phosphonates et des phosphates,
- k est un nombre entier ou fractionnaire, égal ou supérieur à zéro, et
- P est un additif organique,
- a, b, c, d, g et n sont choisis de manière que soit assurée la neutralité électrique du composé (I), c pouvant être égal à 0.
- e, le nombre des molécules de ligand, est 0 ou un nombre entier ou fractionnaire supérieur à 0,
- f et h représentent indépendamment l'un de l'autre 0 ou un nombre entier ou fractionnaire supérieur à 0,
**caractérisé en ce que** pendant la phase d'induction la somme de la pression partielle de gaz inerte et de la pression partielle d'oxyde d'éthylène vaut de 3,0 bars à 6,0 bars et le composé de départ est un alcool de Guerbet.

2. Procédé selon la revendication 1, **caractérisé en ce que** la pression totale au cours de la réaction ne s'élève pas à plus de 11 bars.

3. Procédé selon la revendication 1 ou 2, **caractérisé en ce qu'**au moins l'une des caractéristiques suivantes est réalisée :
(1) M¹ est choisi dans le groupe constitué par Zn²⁺, Fe²⁺, Fe³⁺, Co³⁺, Ni²⁺, Mn²⁺, Co²⁺;
(2) M² est choisi dans le groupe constitué par Fe²⁺, Fe³⁺, CO³⁺.

4. Procédé selon l'une quelconque des revendications 1 à 3, **caractérisé en ce que** M¹ est Zn²⁺ et M² est Co³⁺.

5. Procédé selon l'une quelconque des revendications 1 à 4, **caractérisé en ce que** le composé cyanure bimétallique utilisé en tant que catalyseur est cristallin.

6. Procédé selon l'une quelconque des revendications 1 à 5, **caractérisé en ce que** le mélange d'oxydes d'alkylène présente une teneur en oxyde d'éthylène de plus de 99 %.
